# EUROPEAN PATENT SPECIFICATION

(11) **EP 3 598 113 B1**
(45) Date of publication and mention of the grant of the patent: **22.06.2022**
(21) Application number: 18768688.6
(22) Date of filing: 01.03.2018
(51) Int. Cl.: G01N 22/00, G01N 22/02, G01N 33/02

(54) **NON-DESTRUCTIVE DETECTION METHOD, NON-DESTRUCTIVE DETECTION DEVICES, AND NON-DESTRUCTIVE DETECTION PROGRAM**
ZERSTÖRUNGSFREIES DETEKTIONSVERFAHREN, ZERSTÖRUNGSFREIE DETEKTIONSVORRICHTUNGEN UND ZERSTÖRUNGSFREIES DETEKTIONSPROGRAMM
PROCÉDÉ DE DÉTECTION NON DESTRUCTIF, DISPOSITIFS DE DÉTECTION NON DESTRUCTIF ET PROGRAMME DE DÉTECTION NON DESTRUCTIF

(30) Priority: 17.03.2017 JP 2017053063
(43) Date of publication of application: 22.01.2020
(73) Proprietor: National Institute of Advanced Industrial Science and Technology, Tokyo 100-8921 (JP)
(72) Inventor: KON, Seitaro, Tsukuba-shi, Ibaraki 305-8563 (JP); HORIBE, Masahiro, Tsukuba-shi, Ibaraki 305-8563 (JP); NAGAISHI, Hiroshi, Sapporo-shi, Hokkaido 062-8517 (JP)
(74) Representative: Hoffmann Eitle
(86) International application number: PCT/JP2018/007807
(87) International publication number: WO 2018/168499

(56) References cited:
- WO-A1-01/20311
- JP-A- 2002 522 786
- JP-A- 2007 212 476
- JP-A- 2008 164 594
- JP-A- 2015 161 597
- JP-A- 2015 161 597
- US-A1- 2017 038 311

## Description

### [FIELD]

The present invention relates to a non-destructive detection method, non-destructive detection devices and a non-destructive detection program, and in particular relates to the non-destructive detection method, the non-destructive detection devices and the non-destructive detection program to detect with no damage a foreign substance mixed in a test object such as food.

### [BACKGROUND]

When food is manufactured or harvested with a foreign substance which should not be mixed under ordinary circumstances in the food such as a metal piece, a plastic piece, a glass piece, a stone, soil, hair, etc., and also if such a food product is found after its distribution to consumers, a manufacturer needs to recall a large number of products which causes a large loss and detracts from credibility of the manufacturer of the food product (hereinafter, for convenience, the food product is referred to as simply "food" in the present disclosure). Therefore, quality control in how to detect and eliminate the foreign substance mixed in the food has been a vital issue in food industry. For this reason, non- destructive detection methods for foreign substance in food such as visual detection by human, conventionally, color-sorting method with light, metal detector, X-ray detection method for foreign substance in which image processing technology is combined with high transparency of X-ray have been developed and put to practical use (e.g., Patent Literatures 1, 2 and 3).

Among these, in the color-sorting method using light such as infrared ray, near-infrared ray, visible ray, ultraviolet ray, etc., the foreign substance is detected by irradiating light to the test food, measuring by a receiving light part intensity of reflected light, transmitted light and scattered light of the irradiated light, and comparing with a reference value. Now, since the reference value used in this method is different from for each foreign substance, it is necessary to prepare in advance the reference value and a calibration line for each foreign substance. Therefore, in the color-sorting method, it is necessary to determine in advance an object to be detected as the foreign substance, and the reference value and the calibration line have to be prepared in advance for each foreign substance. In addition, even when the foreign substances belong to the same body, if color of the surface of the foreign substances are different from each other, it is necessary to separately prepare the reference value and the calibration line because of absorptivity of light are different from each other. Therefore, in the color-sorting method, if the object to be detected is unknown, it is extremely difficult to detect it as the foreign substance.

Patent Literature 1 discloses the non-destructive detection method in which the foreign substance such as plastics other than buried metal in the food is detected with no damage by measuring and comparing the intensity of transmitting light or scattered light obtained by irradiating food with laser light in the near infrared wavelength region. In this non-destructive detection method, it is necessary to control the laser light with an optical component such as a lens or to use some means such as to move the test object under a fixed light source by mechanical control. Patent Literature 2 discloses the non-destructive detection method in which the food to be detected is captured with a camera etc., and then it is judged whether a non-metal foreign substance such as hair is mixed in the food by visual check or image recognition based on absorptivity of the captured light. According to this non-destructive detection method, it is possible to search the foreign substance on the test object over a wide range since this method is able to take the test object facially as compared with each of the non-destructive detection methods described above.

Patent Literature 3 discloses a method for executing non-destructive inspection of the test object using X-ray. Since X-ray has high material permeability, this non-destructive detection method using X-ray is an effective method for non-destructive detection of the foreign substance buried in the food as the test object. Furthermore, a non-destructive detection device by radio waves with an antenna are conventionally known (e.g., Non-Patent Literatures 1 and 2 and Patent Literature 4). In these non-destructive detection devices using the radio wave, the non-destructive detection is performed by irradiating the radio wave with an antenna etc., to a measurement object, measuring reflection or transmission and comparing the measured value with a reference value.

Patent Literature 5 discloses the use of an amplitude/phase angle slope to determine moisture content in vegetables after calibration, without mentioning foreign matter. A calibration is required by other means.

Patent Literature 6 discloses the analysis of the change of the dielectric constant of a material to detect foreign objects. The amplitude/phase angle slope is not used, and multiple frequencies are required.

Patent literature 7 discloses the use of phase shift to detect foreign materials in food, without the combined use of amplitude/phase angle slope.

### [Citation List]

### [Patent Literature]

[PTL 1] JP2005-233724A
[PTL 2] JP2014-160013A
[PTL 3] JP2013-130392A
[PTL 4] JP2016-125840A
[PTL 5] JP 2015 161597
[PTL 6] WO 01/20311
[PTL 7] US 2017/038311

### [Non Patent Literature]

[NPL 1] Karim Mazouni et al., "76.5GHz millimeter-wave radar for foreign objects debris detection on airport runways", INTERNATIONAL JOURNAL OF MICROWAVE AND WIRELESS TECHNOLOGIES, vol.4, p.3 (317-326), JUN 2012
[NPL 2] Armin Zeitler et al., "Folded Reflectarrays With Shaped Beam Pattern for Foreign Object Debris Detection on Runways", IEEE TRANSACTIONS ON ANTENNAS AND PROPAGATION, vol.58, p.9 (3065-3068), SEP 2010

### [SUMMARY]

### [Technical Problem]

In any of the non-destructive detection methods using the light described above, when the food as the test object is small in size and is not so thick and also is consisted of material which absorbs less light, it is possible to detect the foreign substance buried in the food by devising the light source or device or by developing the detector ultrahigh sensitively, since light penetrates slightly from the surface of the food into its interior. However, it is hard to detect the foreign substance buried in the food when the test object is a water-rich food with high optical loss, fruit and vegetable with thick skin, or a packaged food that is wrapped and also is light-impermeable. It is also difficult to detect the foreign substance with light when the test object which is cloudy or muddy, for example.

According to the non-destructive detection method using X-ray as described in Patent Literature 3, it is possible to detective relatively easily the foreign substance consisted of metals or materials with high density. However, for the foreign substance consisted of materials having high permeability to X-ray such as a plastic piece, it is extremely difficult to detect since X-ray image of the foreign substance almost integrates with the X-ray image of circumference thereof.

On the other hand, in manufacturing scenes of test objects etc., since radio waves are not easy to narrow down the beam range like light, the radio waves radiated into space are affected by reflections from metals such as surrounding machines. Therefore, in the conventional non-destructive device using the radio waves, in order to distinguish the influence on the foreign substance with the influence on the surroundings, it is necessary to obtain in advance a reference value by measuring property of the radio waves on the object to be detected (i.e., the foreign substance). That is, in this conventional non-destructive device, it is necessary to search in advance transmission property of the radio waves to the foreign substance to be detected.

The present invention has been made in view of the points described above, and an object of the present invention is to provide the non-detection method, the non-destructive detection device and the non-destructive detection program which are able to set a target space easily without searching in advance information on the foreign substance to be detected, and also are able to detect with no damage the foreign substance on the surface of the test object, the foreign substance consisted of metal which is buried in the water-rich test object with high optical loss, and the foreign substance consisted of non-metal such as the plastic piece.

Another object of the present invention is to provide the non-detection method, the non-destructive detection device and the non-destructive detection program which are able to detect positon of the foreign substance in the test object being consisted of the metal or non-metal and also being buried in the test object.

### [Solution to Problem]

In order to achieve the object described above, the present invention discloses a method according to claim 1, devices according to claims 3, 5, 6 and a software according to claim 8.

### [Advantageous Effects of Invention]

According to the present invention, it is possible to detect with no damage not only the foreign substance on the surface of the test object but also the foreign substance consisted of metal which is buried in the water-rich test object with high optical loss and the foreign substance consisted of non-metal such as the plastic piece, while being possible to set the target space easily without having to examine information on the target foreign substance in advance.

### [BRIEF DESCRIPTION of DRAWINGS]

FIG. 1 is a block diagram of one embodiment of a non-destructive detection device according to the present invention;
FIG. 2 is a flowchart for explaining the operation of FIG. 1;
FIG. 3 is a flowchart for explaining the operation of another embodiment of the non-destructive detection device according to the present invention;
FIG. 4 is a block diagram of a first modified example of the non-destructive detection device according to the present invention;
FIG. 5 is a flowchart for explaining the operation of FIG. 4;
FIG. 6 is a flowchart for explaining the operation of a second modified example of the non-destructive detection device according to the present invention;
FIG. 7 is a flowchart for explaining a method of removing drift of a transceiver and a transmission line by looping of the entire non-destructive detection process of a foreign substance;
FIG. 8 is a graph showing an example of results of demonstration experiments;
FIG. 9 is an external view of a fresh salad in a cup container used for a detection experiment; and
FIG. 10 is a graph showing an example of the result of the detection experiment.

### [DESCRIPTION of EMBODIMENTS]

First, principle of the non-destructive detection of the present invention will be described.

In the present invention, an electromagnetic field is generated in a space around a transmission line by inputting a high-frequency signal into the transmission line and transmitting it through the transmission line, and when a test object is brought close to the electromagnetic field, values of a real part (e.g., phase) and an imaginary part (e.g., amplitude), which are expressed by complex notation, of the high-frequency signal which is output from the transmission line are changed in accordance with an electronic property of the test object (specifically, complex dielectric constant). In a 2D position coordinate whose horizontal axis is one of the real part and the imaginary part and vertical axis is the other, values indicating ratios of each variation of the real part and the imaginary part are expressed by a straight line which is changed in accordance with a relative position or a relative distance between the transmission line and the test object (i.e., an amount of transparent electromagnetic field of the test object). Further, a gradient of the straight line changes in accordance with the electronic property of the test object whereas it does not change when the electronic property is constant. On the other hand, the electronic property of the test object in a case where the foreign substance is mixed differs from that in a case where it is mixed.

Therefore, in the present invention, the gradient of the straight line is prepared as a reference for comparison which indicates distribution state of the ratio of each variation of the real part and the imaginary part of the high-frequency signal in the 2D position coordinate which are expressed by complex notation and also obtained when the test object with no foreign substance is transmitted through the electromagnetic field generated in the space around the transmission line. And the reference for comparison is compared with the gradient of the straight line which indicates distribution state of the ratio of each variation of the real part and the imaginary part of the high-frequency signal in the 2D position coordinate which are obtained in the same manner as the way described above for an actual test object whose product tampering is unknown. And it is detected that the foreign substance is mixed in the actual test object when the reference for comparison is different from the gradient of the actual test object. Note that, in the present disclosure, "variation" means a variation of an output high-frequency signal to an input high-frequency signal of the transmission line.

Next, the embodiment of the present invention will be explained.

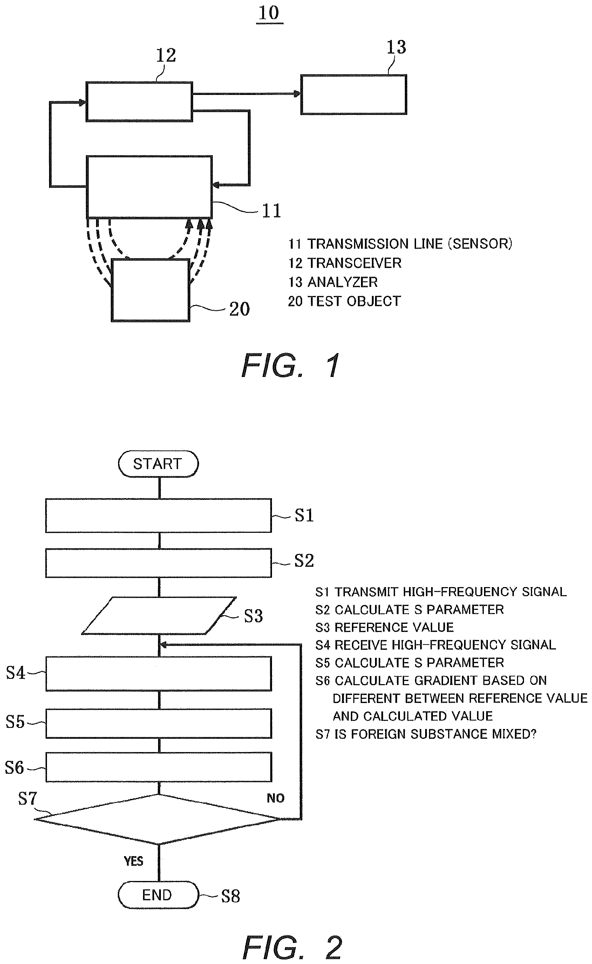

FIG. 1 shows a block diagram of one embodiment of the non-destructive detection device according to the present invention. In the figure, a non-destructive detection device 10 of the present embodiment includes a transmission line 11, a transceiver 12 and an analyzer 13. The transmission line 11 is configured by, for example, a microstripline which generates an electromagnetic field of microwave band in an external surrounding space when, for example, microwave band high-frequency signal is transmitted from its input terminal to its output terminal. The transceiver 12 is configured by, for example, a vector network analyzer (VNA), and has a function to transmit the microwave band high-frequency signal. The analyzer 13 is configured by a computer and a display device etc., and has a function to measure a S parameter (in the present disclosure, specifically, the S parameter is a transmission coefficient indicating voltage ratio of output signal to input signal), analyze the variation of the real part (e.g., phase) and the variation of the imaginary part (e.g., amplitude) of the S parameter and obtain the electronic property of the test object (in this embodiment, the test object is a food).

Next, an operation of the non-destructive detection device 10 of the present embodiment will be described with reference to a flowchart shown in FIG 2. First, the transceiver 12 generates the microwave band high-frequency signal and supplies it to the input terminal of transmission line 11. The transmission line 11 transmits the high-frequency signal supplied to the input terminal according to a design value and outputs it from the output terminal. The transceiver 12 receives the high-frequency signal output from the transmission line 11 (step S1).

The transmission line 11 generates an electromagnetic field distribution in the space around the outside when the high-frequency signal is transmitted. When a test object 20 such as a food sample is brought close to the transmission line 11, the high-frequency signal output from transmission line 11 is attenuated according to the electronic field distribution on the transmission line 11 and the electronic property of the test object 20. Then, value of the real part (e.g., phase) and a value of the imaginary part (e.g., amplitude), which are expressed by complex notation, of the high-frequency signal change in comparison to a case where the test object 20 is absent.

From the transceiver 12 to the analyzer 13, the output high-frequency signal of the transmission line 11 which is received by the transceiver 12 is supplied. The analyzer 13 calculates the S parameter of the high-frequency signal which is supplied firstly to the analyzer 13, analyzes a correspondence between the values of the real part and the imaginary part of the same S parameter (a ratio between the real part and the imaginary part) and set the correspondence as a reference value (steps S2 and S3). Subsequently, the test object 20 is moved while the transceiver 12 receives the high-frequency signal transmitted by the transmission line 11 in the same manner as the way described above (step S4). The analyzer 13 calculates the values (measured values) of the real part and the imaginary part of the S parameter of the high-frequency signal which is received secondly by the transceiver 12 and supplied to the analyzer 13 (step S5), and calculates a gradient based on a difference between the reference value set in the step S3 and the measured value (step S6).

In other words, when the test object 20 moves toward a direction in which the amount of the electromagnetic field transmitting through the test object 20 changes in the electromagnetic field distribution on the transmission line 11, as compared to a case before the movement of the test object 20, the values (measured values) of the real part and the imaginary part of the S parameter of the high-frequency signal which are output from the transmission line 11 to the transceiver 12 change in accordance with the movement. Therefore, the analyzer 13 calculates the gradient of a straight line indicating the distribution state of ratios of the variations which is expressed in the 2D position coordinate in which one of x-axis and y-axis corresponds to the variation of the real part and the other axis corresponds to the variation of the imaginary part (step S6). In such a way, it is possible to eliminate influences of drift in the device system due to surrounding environment such as temperature and humidity, and it is possible to detect the foreign substance even if it is buried in a large loss food etc., since the measured value of the test object (e.g., food etc.) with no foreign substance is used as the reference value. However, if physical property of the food as the test object is significantly different from that of the foreign substance, it is possible to detect the foreign substance easily even if the measured value itself is used without executing the difference processing.

Subsequently, the analyzer 13 judges whether or not the foreign substance is mixed in the test object 20 by comparing the calculated gradient and a reference gradient for comparison which was previously calculated and stored, in the same manner as the way described above, for the test object with no foreign substance (step S7). Here, each measured value of the variations of the real part and the imaginary part changes in accordance with the amount of electromagnetic field that changes when the test object 20 transmits the electromagnetic field distribution on the transmission line 11. However, if the electronic property of test object 20 is common, the ratio of the variation indicated by each measured value (the real part and the imaginary part of the S parameter) in multiple measurements does not depend on size and shape of the test object 20 but distributes on the same line with the same gradient. On the other hand, the reference gradient for comparison is the gradient of the straight line indicating the distribution of the ratio of the variation of the real part and the variation of the imaginary part obtained by measuring in the same manner as the way described above for known test object in which no foreign substance is confirmed to be mixed.

On the other hand, when the foreign substance is on the surface of the test object 20 or it is mixed in the test object 20, the electronic property of the test object 20 is different from the electronic property of the test object 20 in which no foreign substance is mixed. Then, the gradient of the line indicating the distribution of the ratio of the variation of the real part to the variation of the imaginary part is different from the reference gradient for comparison above. Therefore, by judging in the step S7 whether the gradient of the straight line indicating the distribution of the variation indicated by the measured value of the test object 20 is different from the reference gradient for comparison, the analyzer 13 is able to obtain a detection result that the foreign substance is mixed in the test object 20. When it is judged in the step S7 that the foreign substance is not mixed in the test object 20, the analyzer 13 returns to the processing of the steps from S4 to S7 and executes foreign substance judgement based on the next measured value in the same manner as the way described above. And when it is judged in the step S7 that the foreign substance is mixed in the test object 20, the non-destructive detection processing is ended (step S8).

As described above, according to the present embodiment, the straight line indicating the distribution of the ratio of each variation of the real part and the imaginary part of the output high-frequency signal of the transmission line 11 based on the values measured when the test object 20 whose product tampering is unknown is close to the transmission line 11 is compared with the gradient of the straight line, as the comparison reference, indicating the distribution of the ratio of each variation of the real part and the imaginary part of the output high-frequency signal of the transmission line 11 based on the values measured when the test object 20 in which no foreign substance is mixed is close to the transmission line 11. Therefore, without examining in advance information on the foreign substance which is supposed to be mixed, it is possible to detect with no damage the product tampering of the test object 20 without creating a calibration line, which was essential in the prior art, based on information for each foreign substance.

In addition, it is possible to detect with no damage the product tampering in an unopened state even when the test object is covered with a packaging film or plastic, a plastic bottle or a cardboard. Further, according to the present embodiment, it is possible to detect the foreign substance regardless of position and state such as not only the surface of the test object but also buried state inside the test object, size and shape of the foreign substance. Further, according to the present embodiment, it is possible to detect with no damage the foreign substance (e.g., plastic pieces, etc.) which is configured by a substrate having high-permeability to X-ray while being varied in a water-rich food with high optical loss such as vegetables.

Further, in the non-destructive detection device using radio waves, it was necessary to check in advance propagation property of the radio waves to the target foreign substance. In this respect, according to the non-destructive detection device 10 of the present embodiment, since change in electronic property (complex dielectric constant) in a detection range space which is determined by the electromagnetic field distribution occurring in a space around the transmission line 11 is detected, it is possible to obtain relative change in the electric property in a target space without checking in advance the propagation property of the radio waves for each foreign substance that is necessary for the non-destructive detection devices using conventional radio waves. Further, according to the non-destructive detection device 10 of the present embodiment, since the transmission line 11 is used as a sensor, the detection target space is able to be limited thereby it is possible to easily avoid problems that occurs in the non-destructive device using a conventional radio wave with an antenna such as separation of information due to mulita-pass or reflections from surroundings.

As another non-destructive detection device similar to the non-destructive detection device 10 of the present embodiment, the patent literature (JP2015-161597A) mentioned in the present application discloses a moisture amount measuring device. According to the moisture amount measuring device disclosed in this patent literature, in order to estimate with no damage moisture amount of a substance based on variation of the amplitude and phase of the electromagnetic wave obtained by irradiating the test object with the electromagnetic wave, the gradient of the straight line is calculated for each of multiple substrates having known moisture amount of the target object. The gradient of the straight line is obtained in advance by linear approximation of the relationship between the variation of the amplitude and phase of the electromagnetic wave. Further, according to the moisture amount measuring device, the calibration line indicating a relationship between the moisture amount and the gradient of the straight line is calculated in advance with another method such as dry weight method. Then, the moisture amount measuring device calculates the gradient of the straight line indicating the relationship between the variation of the amplitude and phase of the electromagnetic wave obtained by irradiating the test object whose moisture amount is unknown with the electromagnetic wave, judges whether or not the calculated gradient fits any one of the multiple calibration line, and estimates the moisture amount of the test object.

On the other hand, according to the non-destructive detection device 10 of the present embodiment, as described above, the gradient of the straight line is prepared as the reference for comparison which indicates the distribution state of the ratio of each variation of the real part and the imaginary part of the S parameter of the high-frequency signal in the 2D position coordinate which are obtained when the test object with no foreign substance is transmitted through the electromagnetic field generated in the space around the transmission line. And the reference for comparison is compared with the gradient of the straight line which indicates the distribution state of the ratio of each variation of the real part and the imaginary part of the S parameter of the high-frequency signal in the 2D position coordinate which are obtained in the same manner as the way described above for the actual test object whose product tampering is unknown. And it is detected that the foreign substance is mixed in the actual test object when the reference for comparison is different from the gradient of the actual test object. Therefore, according to the non-destructive detection device of the present embodiment, unlike the moisture amount measuring device described above, a unique effect is provided that it is unnecessary to create the calibration line based on the information for each foreign substance.

And now, in the embodiment described above, the gradient of the straight line indicating the distribution of the ratio of the variation of the real part and the variation of the imaginary part of the output high-frequency signal expressed by complex notation is calculated, by moving the position of the position of test object 20 on transmission line 11, based on the measured value of the high-frequency signal transmitting trough the transmission line 11 based on the change in the amount of electromagnetic field of the test object 20 received from the electromagnetic field on the transmission line 11. The reason for the measurement while moving the test object 20 on the transmission line 11 is to change the amount of transparent of the test object on the electromagnetic field in the space around the transmission line 11. In other words, when the test object is not moved but just placed on the transmission line 11, even if multiple measurement are performed, multiple measurement points would overlap at the same coordinate point of the 2D position coordinate and never obtain the gradient of the straight line. However, when the test object 20 is moved on the transmission line 11, since the values of the real part and the imaginary part of the high-frequency signal, which are expressed by complex notation and are output from the transmission line 11, changes, it is possible to obtain the gradient of the straight line indicating the distribution state of the ratio of these variation of the real part and the imaginary part.

On the other hand, in another embodiment of the present invention which will be described next, the test object is just placed on the transmission line and the measurement is performed without moving. The configuration of the present embodiment is almost the same as the configuration shown in the block diagram of FIG. 1. However, unlike the transceiver 12 of FIG. 1, in the present embodiment, the transceiver is configured to transmit through the transmission line a high-frequency signal at an optimum frequency for the transceiver to detect the foreign substance of the test object and the high-frequency signal swept at a predetermined frequency range of about 10% to 20% around the same optimum frequency, or transmit through the transmission line a synthetic high-frequency signal consisting of the optimum frequency element and multiple frequency elements within the frequency range.

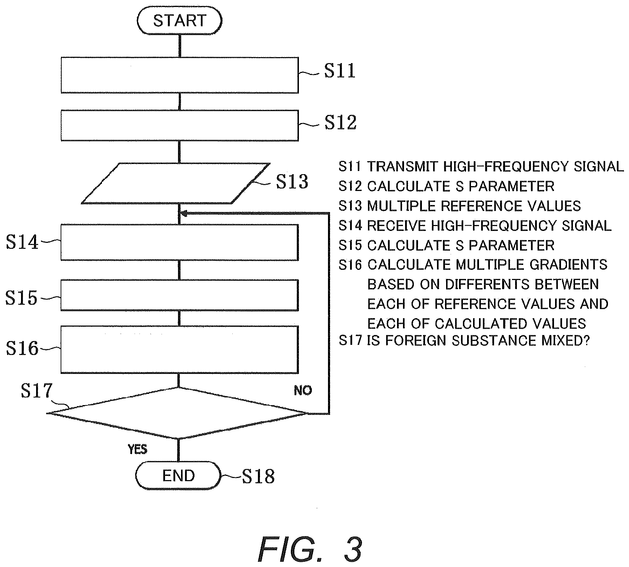

The present embodiment will be described with reference to the flowchart shown in FIG. 3. The transmission line generates its surrounding the electromagnetic field corresponding to each of the elements of the optimum frequency and surrounding multiple frequency of the high-frequency signal supplied from the transceiver described above, and outputs the high-frequency signal indicating the variation in accordance with multiple different electronic properties of the same test object for each element of the optimum frequency element and the surrounding multiple frequency elements. The transceiver receives the high-frequency signal from the transmission line (step S11). The high-frequency signals received by the transceiver are signals having different variations for every of the rear parts or the imaginary parts which indicate the variations in accordance with the multiple different electronic properties of the same test object for each element of the optimum frequency element and the surrounding multiple frequency elements. In such a way, the analyzer calculates each S parameter for each frequency of the high-frequency signals (step S12) and obtains the reference values of the gradients of the multiple straight lines, substantially similar to the embodiment shown in FIG. 1, based on the data of multiple different measurement points indicating each value of the real parts and the imaginary parts of the high-frequency signals (step S13).

Subsequently, the transceiver receives the high-frequency signals transmitted through the transmission line in the same manner as the way described above (step S14). The output high-frequency signal of the transmission line 11 which is secondly received by the transceiver 12 is supplied the analyzer 13, the analyzer 13 calculates the S parameter for each frequency of the high-frequency signal (step S15) and calculates the gradients of the multiple straight lines obtained using differences between the multiple reference values set in the step S13 and each value of the real parts and imaginary parts of the S parameter based on the data of the multiple different measurement points indicating each value of the real parts and the imaginary parts (step S16). In such a way, by the analyzer 13 calculates the gradients of the multiple straight lines indicating the distribution of the ratios of the variations that are expressed in the 2D position coordinate in which one of x-axis and y-axis corresponds to the variation of the real part and the other axis corresponds to the variation of the imaginary part. The analyzer compares the multiple gradients of the straight lines with the single reference gradient for comparison which was previously calculated and stored, in the same manner as the way described above, for the known test object in which no foreign substance is confirmed to be mixed, judges that the foreign substance is not mixed in the test object when all of the multiple gradients of the straight lines match with the reference gradient (step S17), and returns to the processing of the step S14 again. On the other hand, in the step S17, the analyzer judges that the foreign substance is mixed in the test object when any one of the multiple gradients of the straight lines differs from the reference gradient (step S17) and finishes the non-destructive detection processing of the product tampering in the test object (step S18).

Therefore, according to the present embodiment, it is possible to perform the non-destructive detection of the foreign substance mixed in the test object without moving the test object on the transmission line. The present embodiment is able to be applied not only to the non-destructive detection of the foreign substance but also to general quality inspection technology with the transmission line as a sensor in which quality such as moisture amount and sugar content, etc., is measured with no damage.

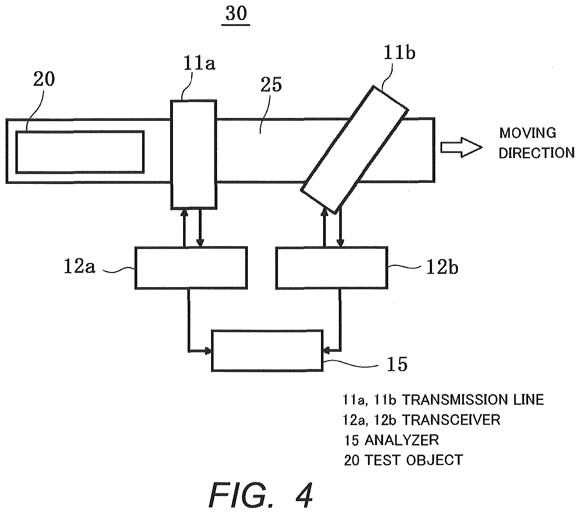

Next, in the non-destructive detection device according to the present invention, in addition to the non-destructive detection of the foreign substance mixed on the surface or inside of the test object, two modified examples for detecting the position of the foreign substance will be described. FIG. 4 is a block diagram of a first modified example of the non-destructive detection device according to the present invention. As shown in the figure, a non-destructive detection device 30 of the first modified example is configured by transmission lines 11a and 11b, transceivers 12a and 12b, and an analyzer 15. In other words, the non-destructive detection device 30 of the modified example has a configuration in which two sets of the transmission line 11 and the transceiver 12 in the non-destructive detection device 10 shown in FIG. 1 are provided. The transmission line 11a has its longitudinal direction arranged orthogonally to the moving direction of a belt conveyor 25. The transmission line 11b is arranged downstream of the transmission line 11a with respect to the moving direction of the belt conveyor 25 and its longitudinal direction is arranged obliquely. The test object 20 is placed on the belt conveyor 25 so as to move at a known constant speed over an upper position of the electromagnetic field distribution of the transmission lines 11a and 11b.

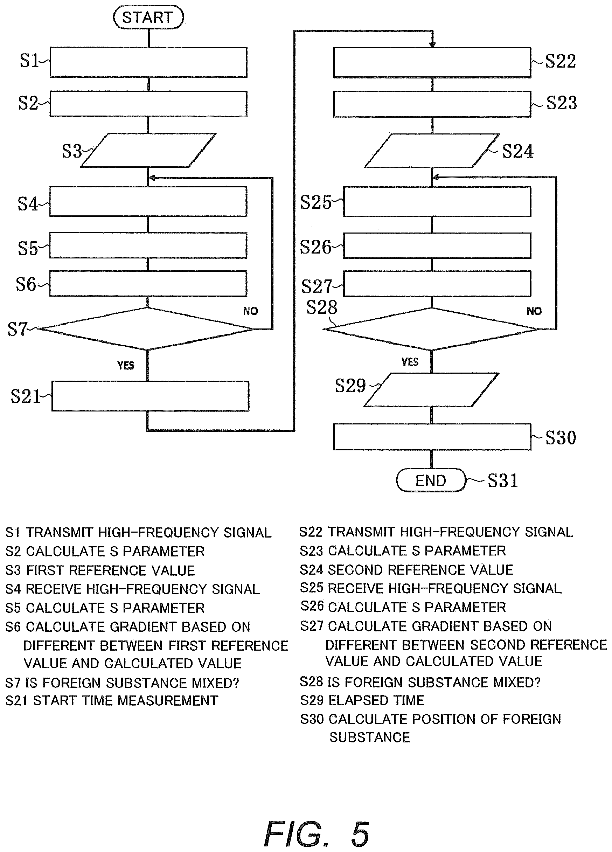

Next, the operation of the non-destructive detection device 30 of the modified example will be described with reference to the flowchart shown in FIG. 5. In FIG. 5, the same processing steps as those in FIG. 2 are assigned the same signs and the explanation thereof is omitted. First, based on the high-frequency signal which is transmitted from the transceiver 12a, transmitted through the transmission line 11a, received by the transceiver 12a and supplied to the analyzer 15, the analyzer 15 calculates the S parameter of the high-frequency signal transmitted through the transmission line 11a and execute the gradient calculation based on the difference between the reference value set in the step S3 shown in FIG. 5 and the measured value (steps S5 and S6). The analyzer 15 calculates the gradient of the straight line indicating the distribution state of ratios of the variations which is expressed in the 2D position coordinate in which one of x-axis and y-axis corresponds to the variation of the real part and the other axis corresponds to the variation of the imaginary part (step S6 described above) and judges whether or not the foreign substance is mixed in the test object 20 by comparing the calculated gradient and the reference gradient for comparison which was previously calculated and stored, using the transmission line 11a, in the same manner as the way described above, for the test object with no foreign substance (step S7 shown in FIG. 5).

When the analyzer 15 judges that the foreign substance is present because the gradient of the straight line indicating the distribution of the ratio of the variation indicated by the measured value of test object 20 in the step S7 shown in FIG. 5 is different from a first reference gradient for comparison, it performs following operation. First, the analyzer 15 starts time measurement (step S21). Subsequently, based on the high-frequency signal which is transmitted from the transceiver 12b, transmitted through the transmission line 11b, received by the transceiver 12b and supplied firstly to the analyzer 15, the analyzer 15 calculates the S parameter of the high-frequency signal transmitted through the transmission line 11b (steps S22 and S23). Subsequently, the analyzer 15 analyzes the correspondence between the values of the real part and the imaginary part of the same S parameter (the ratio between the real part and the imaginary part) and set the correspondence as a second reference value (step S24).

The high-frequency signals are continuously transmitted from the transceiver 12b, received by the transceiver 12b and then input to the analyzer 15 (step S25). The analyzer 15 calculates the S parameter of the second high-frequency signal which was transmitted through the transmission line 11b and received in the step S25 (step S26), and executes gradient calculation based on the difference between the second reference value and the correspondence between the values of the real part and the imaginary part of the same S parameter (step S27). In other words, the analyzer 15 calculates in the step S27 the gradient of the straight line connecting the measurement point indicating the second reference value with the measurement point indicating the ratio of the real part and the imaginary part of the S parameter which was calculated in the step S26 in the 2D position coordinate in which one of x-axis and y-axis corresponds to the variation of the real part and the other axis corresponds to the variation of the imaginary part.

Subsequently, the analyzer 15 compares the gradient calculated in the step S27 with the reference gradient for comparison and judges whether or not the foreign substance is mixed in the test object 20 (step S28). In the step S28, the analyzer 15 judges that the foreign substance is not mixed when the reference gradient substantially matches the gradient calculated in the step S27, returns to the processing of the step S25, and executes processing to judge whether or not the foreign substance is not mixed. On the other hand, the analyzer 15 judges in the step S28 that the foreign substance is mixed when the reference gradient is different from the calculated gradient described above and calculates elapsed time from start of the time measurement in the step S21 to judgement time at the step S28 (step 29).

The analyzer 15 calculates the position of the foreign substance mixed in the test object 20 based on the elapsed time described above since the speed of the belt conveyor 25 is a known constant speed, the longitudinal direction of the transmission line 11a is arranged orthogonally to the moving direction of a belt conveyor 25 and the transmission line 11b is arranged downstream of the transmission line 11a and the longitudinal direction of the transmission line 11b is arranged obliquely to the moving direction described above (step S30). In other words, the position of the foreign substance is determined based on time gap between the elapsed time and a predetermined movement time of a distance center positions of the transmission lines 11a and 11b in the moving direction of the belt conveyor 25. Then, the analyzer 15 finishes the processing to detect the position of the foreign substance (step S31). Note that it is possible to detect the position of the foreign substance in the same manner as the way described above even when the movement direction of the belt conveyor 25 is opposite to that in the above example, and the transmission line 11b is arranged obliquely and the transmission line 11a is orthogonally arranged downstream of the transmission line 11b.

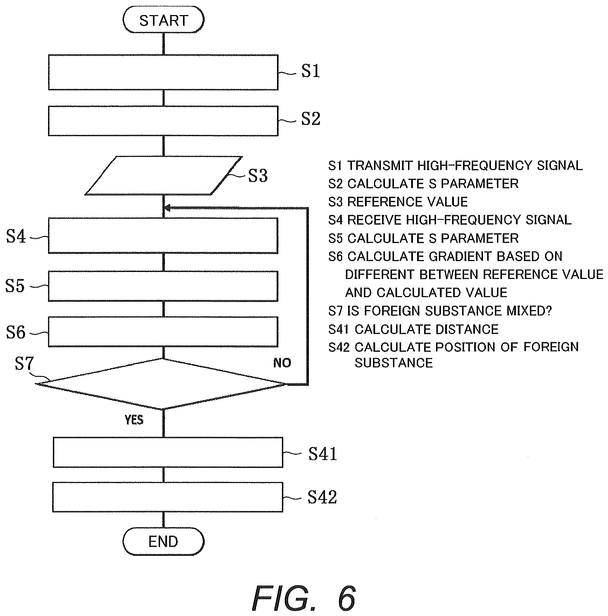

Next, a second modified example will be described in which the position of the foreign substance is detected using time domain measurement. The configuration of this second modified example is similar to the configuration shown in FIG. 1 whereas analysis algorithm of the analyzer 13 is different from the embodiment of FIG. 1. The operation of the non-destructive detection device according to the second modified example will be described with reference to the flowchart shown in FIG. 6. Note that the same processing steps as those in FIG. 2 are assigned the same signs and the explanation thereof is omitted. When it is judged in the step S7 that the foreign substance is mixed, the analyzer 13 performs measurement of time domain which is measured by mathematical transformation into time domain waveform from waveform obtained within the frequency domain in the step S4 etc., and detect amplitude and position of mismatch that appears when the foreign substance is mixed in the test object 20 on the transmission line 11.

In other words, the analyzer 13 is configured by a VNA and measures time response of the high-frequency signal which is transmitted by the transceiver 12 by calculating the S parameter including not only the input signal and output signal of the transmission line 11 but also reflected signal which is reflected and supplied to the transceiver 12, thereby the analyzer 13 calculates a distance based on a difference in time responses between the position where the foreign substance is present and the position where the foreign substance is absent in the test object 20 that was judged to have the foreign substance on the transmission line 11 (step S41). Subsequently, the analyzer 13 determines the position at which the foreign substance is mixed in the test object 20 based on the distance which was calculated with the difference in time response of the high-frequency signal (step S42). Note that when the measured value which was obtained by using high speed sweep based on digital sweep is analyzed by the analyzer 13, it is possible to determine at high speed at which the foreign substance is present inside the test object 20 on the transmission line 11.

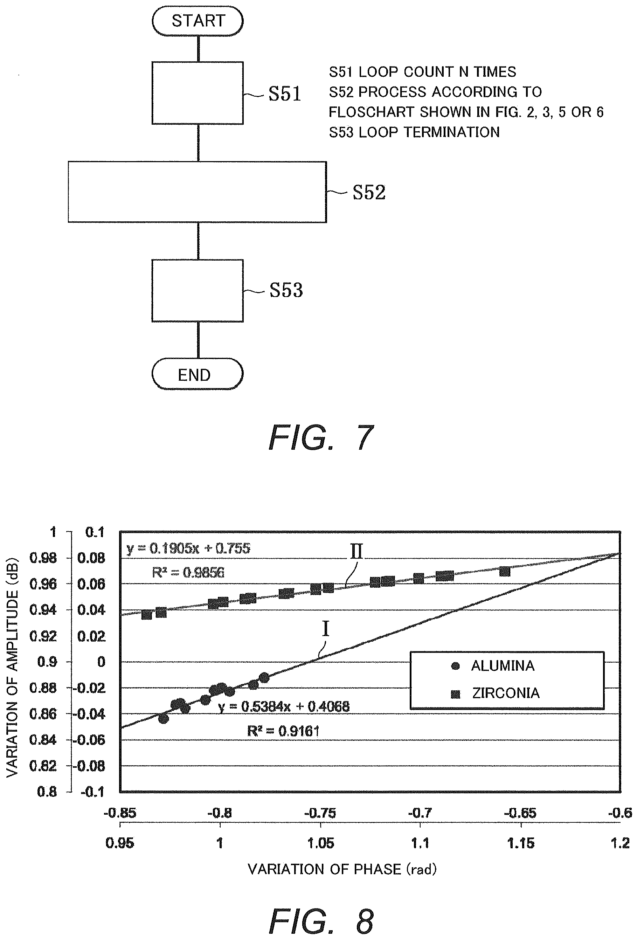

Next, with reference to the flowchart shown in FIG. 7, a method of removing drift of the transceiver and the transmission line by looping of the entire non-destructive detection process of the foreign substance. When the foreign substance detection method according to the flowcharts shown in FIG. 2, FIG. 3, FIG. 5 and FIG. 6 is executed for a long time, influences caused by the drift of the transceiver and the transmission line may appear due to influences of surrounding environment. In order to maintain high detection sensitivity, it is desirable to obtain the reference value periodically. Therefore, the foreign substance detection process according to the flowchart shown in FIG. 2, FIG. 3, FIG. 5 or FIG. 6 is periodically repeated N times (N is a desired natural number) and executed in a loop to reset the reference value every N times (steps S51 to S53 shown in FIG. 7). As such, it is possible to maintain the detection sensitivity in a high state.

And now, when the thickness or width of the food as the test object is large and the foreign substance mixed in the food is located away from the transmission line, it is difficult to perform the non-destructive detection of the foreign substance. Therefore, as measures against this problem, two transmission lines are arranged to locate away from one another so as to sandwich the test object between downward and upward or right and left. As such, even when the foreign substance exists at a distance from one of the transmission lines, it is possible for the other transmission line to detect the foreign substance with high sensitivity because the distance from the other transmission line is short. When the food passes inside a pipe etc. having a roll shape such as a cylindrical shape, it is possible to detect the foreign substance with high sensitivity by preparing the transmission line with a flexible material and printing technology etc., and arranging the transmission line to fit the inner shape of the pipe.

Next, results of a demonstration experiment of the non-destructive detection device 10 of the present embodiment will be described. First, in order to demonstrate the principle of the foreign substance detection, demonstration experiment for principle was conducted by preparing two types of ceramic samples with known electronic properties and different sizes. According to the principle of the present embodiment, if the electronic properties are the same, the measured values (the values of the real part and the imaginary part of the high-frequency signal) should be found on the same straight line in the 2D position coordinate indicating the ratio of the real part and the imaginary part of the high-frequency signal regardless of the size and shape of the test object. Therefore, alumina samples with particle sizes of 1.5 mm, 3 mm and 5 mm and zirconia samples with particle sizes of 3 mm and 5 mm were used.

FIG. 8 is a graph showing the results of the demonstration experiment described above. In the figure, the horizontal axis represents the variation (rad in unit) of the phase which was calculated based on the value of the real part of the received high-frequency signal and the value of the imaginary part of the received high-frequency signal, and the vertical axis represents the variation (dB in unit) of the amplitude which was calculated based on the value of the real part of the received high-frequency signal and the value of the imaginary part of the received high-frequency signal. Further, in FIG. 8, in the numerical values shown in the two columns of the vertical axis and the numerical values shown in the two rows of the horizontal axis, the numerical values shown inside of the graph and close to the graph are those of the alumina samples, whereas the numerical values shown outside of the graph are those of the zirconia samples. In the 2D position coordinate graph shown in FIG. 8, it was confirmed that the ratios of the variations of the phase and the variations of the amplitude of the alumina samples are distributed on the straight line shown by I and the ratios of the variations of the phase and the variations of the amplitude of the zirconia samples are distributed on the straight line shown by II. It was also confirmed that the gradients of the straight lines I of the alumina samples and the straight line II of the zirconia sample which are different in the electric property from each other were different values in accordance with the electric property.

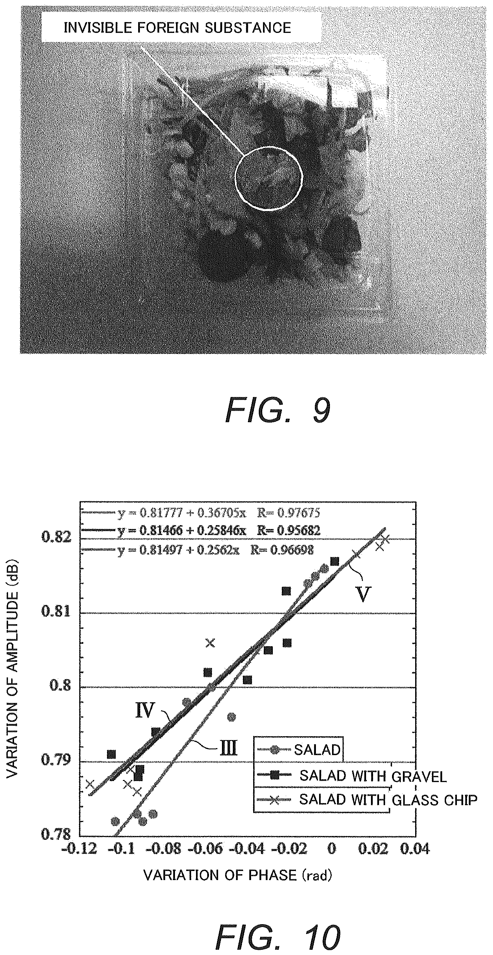

Next, an example in which the foreign substance mixed in food actually is detected will be described. The test object is a fresh salad in a cup, which is commercially available at convenience stores, supermarkets, etc., whose appearance is shown in FIG. 9. The foreign substance was put in a position which was not visible from the outside in this salad, and a detection experiment of the foreign substance based on the present embodiment was carried out through the cup of the fresh salad with cap. FIG. 10 is a graph showing the result of the experiment. In the figure, the horizontal axis represents the variation (rad in unit) of the phase which was calculated based on the value of the real part of the received high-frequency signal and the value of the imaginary part of the received high-frequency signal, and the vertical axis represents the variation (dB in unit) of the amplitude which was calculated based on the value of the real part of the received high-frequency signal and the value of the imaginary part of the received high-frequency signal.

In FIG. 10, the straight line III in which a plurality of measurement points shown with circle lie alongside indicates the property of the fresh salad with cap in which the foreign substance is not mixed. The straight line IV in which a plurality of measurement points shown with box lie alongside indicates the property of the fresh salad with cap in which a gravel is mixed as the foreign substance. The straight line V in which a plurality of measurement points shown with cross lie alongside indicates the property of the fresh salad with cap in which a glass chip is mixed as the foreign substance. As can be seen from FIG. 10, since the straight lines IV and V indicating the properties of the foods (fresh salad with cup) in which the foreign substance is mixed are different in the gradient from the straight line III indicating the property of the food in which the foreign substance is not mixed, it is possible to detect the product tampering with no damage. In the same manner, it has been confirmed that the detection of insects mixed in the food is also possible.

As described above, the non-destructive detection device of the present embodiment or the modified samples is able to be applied to industrial products, their manufacturing materials and irregular samples such as agricultural products and foods and it is possible to detect the foreign substance through the cup on a real-time basis with no damage.

Note that the present invention includes a non-destructive detection program that makes a computer as the analyzer execute the processing steps of the non-destructive detection method. The non-destructive detection program may be reproduced from a recording medium and loaded into an execution memory of the computer, or may be distributed to a communication network and downloaded to the computer.

### [Reference Signs List]

10, 30 Non-destructive detection device
11, 11a, 11b Transmission line
12, 12a, 12b Transceiver
13, 15 Analyzer
20 Test object
25 Belt Conveyor

## Claims

1. A non-destructive detection method, comprising:
signal transmission step (S1) of transmitting a microwave band constant high-frequency signal to a transmission line (11) and generating outside of the transmission line (11) an electromagnetic field at a position where a test object (20) is at least present;
reference measurement step (S2, S3) of measuring the microwave band constant high-frequency signal transmitted through the transmission line (11) and setting a value which is measured firstly as a reference value;
measurement step (S4, S5) of measuring the microwave band constant high-frequency signal transmitted through the transmission line (11) while moving the position of the test object (20) and obtaining a value which is measured secondly or later;
gradient calculation step (S6) of calculating a gradient of a straight line at a predetermined 2D position coordinate based on a difference between the reference value and the value which is measured secondly or later, wherein the predetermined 2D position coordinate is a 2D position coordinate whose vertical axis is one of real and imaginary parts of a S parameter of the microwave band constant high-frequency signal expressed by complex notation or one of phase and amplitude obtained by calculating the real and imaginary parts and whose horizontal axis is the other of the real and imaginary parts or the other of the phase and amplitude, wherein the S parameter is a transmission coefficient indicating voltage ratio of output signal to input signal; and
judgment step (S7) comprising the steps of:
comparing a reference gradient with the gradient which is calculated in the gradient calculation step, the reference gradient is the gradient of the straight line at the predetermined 2D position coordinate which was calculated in advance based on the measured value of the microwave band constant high-frequency signal from the transmission line (11) when the microwave band constant high-frequency signal is transmitted to the transmission line (11) and also the electromagnetic field of the transmission line (11) is generated at a position where a known test object exists, the known test object was confirmed in advance that no foreign substance is mixed; and
when the reference gradient is different from the calculated gradient, judging that a foreign substance is mixed in the test object (20).

2. The non-destructive detection method according to claim 1,
wherein the values measured at the reference value measuring step (S2, S3) and the measuring step (S4, S5), and the measured value of the microwave band constant high-frequency signal used for the calculation of the reference gradient are values of the real and imaginary parts.

3. A non-destructive detection device, comprising:
a transmission line (11);
transceiver means (12) which is configured to:
transmit a microwave band constant high-frequency signal to the transmission line (11);
generate outside of the transmission line (11) an electromagnetic field; and
receive the microwave band constant high-frequency signal output from the transmission line (11) ;
measurement means (12) which is configured to:
measure the microwave band constant high-frequency signal transmitted through the transmission line (11), the device being arranged to simultaneously move a position of a test object (20) within the generated electromagnetic field;
set a value which is measured firstly as a reference value; and
set a value which is measured secondly or later as a measured value for judgment;
gradient calculation means (13) which is configured to calculate a gradient of a straight line at a predetermined 2D position coordinate based on a difference between the reference value and the measured value for judgment, wherein the predetermined 2D position coordinate is a 2D position coordinate whose vertical axis is one of real and imaginary parts of a S parameter of the microwave band constant high-frequency signal expressed by complex notation or one of phase and amplitude obtained by calculating the real and imaginary parts and whose horizontal axis is the other of the real and imaginary parts or the other of the phase and amplitude, wherein the S parameter is a transmission coefficient indicating voltage ratio of output signal to input signal; and
judgment means (13) which is configured to:
compare a reference gradient with the gradient which is calculated by the gradient calculation means (13), the reference gradient is the gradient of the straight line at the predetermined 2D position coordinate which was calculated and memorized in advance based on the measured value of the microwave band constant high-frequency signal from the transmission line (11) when a known test object is arranged within the electromagnetic field which is generated outside of the transmission line (11) by transmitting the microwave band constant high-frequency signal to the transmission line (11), the known test object was confirmed in advance that no foreign substance is mixed; and
when the reference gradient is different from the calculated gradient, judge that a foreign substance is mixed in the test object (20).

4. The non-destructive detection device according to claim 3, further comprising position specifying means (13), wherein the position specifying means (13) is configured to:
transform into a waveform in a time domain from a waveform obtained in a frequency signal that is transmitted on the transmission line (11) when the test object (20), in which it is judged by the judgment means (13) that the foreign substance is mixed, is arranged within the electromagnetic field of the transmission line (11);
detect an amplitude and position of a mismatch corresponding to the foreign substance, wherein the detection is performed by calculating the S parameter including the input signal and output signal of the transmission line (11), and the reflected signal which is returning to the transceiver (12);
calculate a distance based on a difference in time response between the position where the foreign substance is present and the position where the foreign substance is absent in the test object (20) that was judged to have the foreign substance on the transmission line (11); and
determine the position at which the foreign substance is mixed in the test object (20) based on the distance which was calculated with the difference in time response of the high-frequency signal.

5. A non-destructive detection device, comprising:
a first transmission line (11a) whose longitudinal direction is arranged orthogonally to moving direction of a test object (20) in motion;
a second transmission line (11b), which is arranged at downstream or upstream of the moving direction of the test object (20) with respect to the first transmission line (11a), whose longitudinal direction is arranged obliquely to the moving direction of the test object (20);
transmission means (12a, 12b) which is configured to:
generate and transmit a microwave band constant high-frequency signal to the first and second transmission lines (11a, 11b);
generating outside of the transmission line an electromagnetic field; and
receive the microwave band constant high-frequency signal output from the first and second transmission lines (11a, 11b);
first measurement means (12a) which is configured to:
obtain first value obtained by measuring the microwave band constant high-frequency signal which is output from one of the transmission lines (11a, 11b) which is arranged at the upstream between the first and second transmission lines (11a, 11b) and also measured by receiving by the transmission means(12a, 12b), the device being arranged to simultaneously move a position of a test object (20) within the generated electromagnetic field;
set the first value which is measured firstly as a first reference value; and
set the first value which is measured secondly or later as a first measured value for judgment;
first gradient calculation means (15) which is configured to calculate a gradient of a first straight line at a predetermined 2D position coordinate based on a difference between the first reference value and the first measured value for judgement, wherein the predetermined 2D position coordinate is a 2D position coordinate whose vertical axis is one of real and imaginary parts of a S parameter of the microwave band constant high-frequency signal expressed by complex notation or one of phase and amplitude obtained by calculating the real and imaginary parts and whose horizontal axis is the other of the real and imaginary parts or the other of the phase and amplitude, wherein the S parameter is a transmission coefficient indicating voltage ratio of output signal to input signal;
first judgment means (15) which is configured to:
compare a reference gradient with the gradient of the first straight line which is calculated by the first gradient calculation means (15), the reference gradient is the gradient of the straight line at the predetermined 2D position coordinate which was calculated and memorized in advance based on the measured value of the microwave band constant high-frequency signal from the first transmission line (11a) when a known test object is arranged within the electromagnetic field which is generated outside of the first transmission line (11a) by transmitting the microwave band constant high-frequency signal to the first transmission line (11a), the known test object was confirmed in advance that no foreign substance is mixed; and
when the reference gradient is different from the calculated gradient of the first straight line, judge that a foreign substance is mixed in the test object (20);
second measurement means (12b) which is configured to:
obtain second value obtained by measuring the microwave band constant high-frequency signal which is output from one of the transmission lines (11a, 11b) which is arranged at the downstream between the first and second transmission lines (11a, 11b) and also measured by receiving by the transmission means(12a, 12b), the device being arranged to simultaneously move the position of the test object (20) within the generated electromagnetic field;
set the second value which is measured firstly as a second reference value; and
set the second value which is measured secondly or later as a second measured value for judgment;
second gradient calculation means (15) which is configured to calculate a gradient of a second straight line at the predetermined 2D position coordinate based on a difference between the second reference value and the second measured value for judgement;
second judgment means (15) which is configured to:
compare the reference gradient with the calculated gradient of the second straight line; and
when the reference gradient is different from the calculated gradient of the second straight line, judge that the foreign substance is mixed in the test object (20); and
interval measurement means (15) which is configured to measure an interval between timing at which the first judgment means (15) judges that the foreign substance is mixed in the test object (20) and timing at which the second judgment means (15) judges that the foreign substance is mixed in the test object (20),
wherein a position of the foreign substance mixed in the test object (20) is detected with no damage based on the measured interval by the interval measurement means (15) .

6. A non-destructive detection device, comprising:
a transmission line (11); transmission means (12) which is configured to:
generate and transmit to the transmission line (11) a microwave band high-frequency signal or a synthetic high-frequency signal, the high-frequency signal is generated by sweeping with a predetermined frequency range centered on an optimum frequency for detecting a test object (20), the synthetic high-frequency signal consisting of an optimum frequency within the frequency range and other multiple frequency elements;
generate multiple electromagnetic fields corresponding to multiple frequencies of the high-frequency signal, the generated multiple electromagnetic fields extending to a position where the test object (20) can be placed; and
receive the high-frequency signal output from the transmission line (11);
measurement means (12) which is configured to:
measure multiple values corresponding to the multiple frequencies of the high-frequency signal obtained by measuring the high-frequency signal received by the transmission means (12);
set multiple values which are measured firstly as multiple reference values; and
set multiple values which are measured secondly or later as multiple measured values for judgment;
gradient calculation means (13) which is configured to calculate multiple gradients of multiple straight lines at a predetermined 2D position coordinate based on a difference in each frequency between the reference values and the multiple measured values for judgment, wherein the predetermined 2D position coordinate is a 2D position coordinate whose vertical axis is one of real and imaginary parts of a S parameter of the high-frequency signal expressed by complex notation or one of phase and amplitude obtained by calculating the real and imaginary parts and whose horizontal axis is the other of the real and imaginary parts or the other of the phase and amplitude, wherein the S parameter is a transmission coefficient indicating voltage ratio of output signal to input signal; and
judgment means (13) which is configured to:
compare a reference gradient with each of the multiple gradients of the multiple straight lines which are calculated by the gradient calculation means (13), the reference gradient is the gradient of the straight line at the predetermined 2D position coordinate which was calculated and memorized in advance based on the measured value of the high-frequency signal from the transmission line (11) when a known test object is arranged within the electromagnetic field which is generated outside of the transmission line (11) by transmitting the high-frequency signal to the transmission line (11), the known test object was confirmed in advance that no foreign substance is mixed; and
when the reference gradient is different from any one of the calculated multiple gradients, judge that a foreign substance is mixed in the test object (20) .

7. The non-destructive detection device according to any one of claims 3 to 6,
wherein the measured value of the high-frequency signal is value of the real and imaginary parts.

8. A computer program comprising instructions which, when executed by a non-destructive detection device according to any of claims 3 to 5, and claim 7 when dependent on any of claims 3 to 5, cause the non-destructive detection device to execute the non-destructive detection method according to claim 1 or 2.

## Patentansprüche

1. Zerstörungsfreies Detektionsverfahren, umfassend:
Signalübertragungsschritt (S1) des Übertragens eines konstanten Mikrowellenband-Hochfrequenzsignals an eine Übertragungsleitung (11) und Erzeugens eines elektromagnetischen Felds außerhalb der Übertragungsleitung (11) an einer Position, an der ein Prüfobjekt (20) mindestens vorhanden ist;
Referenzmessschritt (S2, S3) des Messens des konstanten Mikrowellenband-Hochfrequenzsignals, das durch die Übertragungsleitung (11) übertragen wird, und Festlegens eines Werts, der zuerst als ein Referenzwert gemessen wird;
Messschritt (S4, S5) des Messens des konstanten Mikrowellenband-Hochfrequenzsignals, das durch die Übertragungsleitung (11) übertragen wird, während die Position des Prüfobjekts (20) bewegt wird, und Erhaltens eines Werts, der an zweiter Stelle oder später gemessen wird;
Gradientenberechnungsschritt (S6) des Berechnens eines Gradienten einer geraden Linie bei einer vorher festgelegten 2D-Positionskoordinate basierend auf einem Unterschied zwischen dem Referenzwert und dem Wert, der an zweiter Stelle oder später gemessen wird, wobei die vorher festgelegte 2D-Positionskoordinate eine 2D-Positionskoordinate ist, deren vertikale Achse eins ist von realem oder imaginärem Teil eines S-Parameters des konstanten Mikrowellenband-Hochfrequenzsignals, ausgedrückt durch komplexe Notation oder eins von Phase und Amplitude, erhalten durch Berechnen des realen und imaginären Teils, und deren horizontale Achse der andere des realen und imaginären Teils oder die andere der Phase und Amplitude ist, wobei der S-Parameter ein Übertragungskoeffizient ist, der Spannungsverhältnis von Ausgangssignal zu Eingangssignal angibt; und
Beurteilungsschritt (S7), umfassend die Schritte des:
Vergleichens eines Referenzgradienten mit dem Gradienten, der in dem Gradientenberechnungsschritt berechnet wird, wobei der Referenzgradient der Gradient der geraden Linie an der vorher festgelegten 2D-Positionskoordinate ist, der im Voraus basierend auf dem gemessenen Wert des konstanten Mikrowellenband-Hochfrequenzsignal aus der Übertragungsleitung (11) berechnet wurde, wenn das konstante Mikrowellenband-Hochfrequenzsignal an die Übertragungsleitung (11) übertragen wird, und auch das elektromagnetische Feld der Übertragungsleitung (11) an einer Position erzeugt wird, an der ein bekanntes Prüfobjekt existiert, wobei das bekannte Prüfobjekt im Voraus bestätigt wurde, dass keine Fremdsubstanz gemischt ist; und
wenn sich der Referenzgradient von dem berechneten Gradienten unterscheidet, Urteilen, dass eine Fremdsubstanz in dem Prüfobjekt (20) gemischt ist.

2. Zerstörungsfreies Detektionsverfahren nach Anspruch 1,
wobei die bei dem Referenzwertmessschritt (S2, S3) und dem Messschritt (S4, S5) gemessenen Werte, und der gemessene Wert des für die Berechnung des Referenzgradienten verwendeten konstanten Mikrowellenband-Hochfrequenzsignals Werte des realen und imaginären Teils sind.

3. Zerstörungsfreie Detektionsvorrichtung, umfassend:
eine Übertragungsleitung (11);
Transceivermittel (12), das konfiguriert ist, um:
ein konstantes Mikrowellenband-Hochfrequenzsignal an die Übertragungsleitung (11) zu übertragen;
außerhalb der Übertragungsleitung (11) ein elektromagnetisches Feld zu erzeugen; und
das von der Übertragungsleitung (11) ausgegebene konstante Mikrowellenband-Hochfrequenzsignal zu empfangen;
Messmittel (12), das konfiguriert ist, um:
das durch die Übertragungsleitung (11) übertragene konstante Mikrowellenband-Hochfrequenzsignal zu messen, wobei die Vorrichtung angeordnet ist, um gleichzeitig eine Position eines Prüfobjekts (20) innerhalb des elektromagnetischen Felds zu bewegen;
einen Wert, der zuerst gemessen wird, als einen Referenzwert festzulegen; und
einen Wert, der an zweiter Stelle oder später gemessen wird, als einen gemessenen Wert zur Beurteilung festzulegen;
Gradientenberechnungsmittel (13), das konfiguriert ist, um einen Gradienten einer geraden Linie bei einer vorher festgelegten 2D-Positionskoordinate basierend auf einem Unterschied zwischen dem Referenzwert und dem gemessenen Wert zur Beurteilung zu berechnen, wobei die vorher festgelegte 2D-Positionskoordinate eine 2D-Positionskoordinate ist, deren vertikale Achse eins ist von realem oder imaginärem Teil eines S-Parameters des konstanten Mikrowellenband-Hochfrequenzsignals, ausgedrückt durch komplexe Notation oder eins von Phase und Amplitude, erhalten durch Berechnen des realen und imaginären Teils, und deren horizontale Achse der andere des realen und imaginären Teils oder die andere der Phase und Amplitude ist, wobei der S-Parameter ein Übertragungskoeffizient ist, der Spannungsverhältnis von Ausgangssignal zu Eingangssignal angibt;
und Beurteilungsmittel (13), das konfiguriert ist, um:
einen Referenzgradienten mit dem Gradienten zu vergleichen, der durch das Gradientenberechnungsmittel (13) berechnet wird, wobei der Referenzgradient der Gradient der geraden Linie an der vorher festgelegten 2D-Positionskoordinate ist, der im Voraus basierend auf dem gemessenen Wert des konstanten Mikrowellen-Hochfrequenzsignals von der Übertragungslinie (11) berechnet und gespeichert wurde, wenn ein bekanntes Prüfobjekt innerhalb des elektromagnetischen Felds angeordnet ist, das außerhalb der Übertragungsleitung (11) durch Übertragen des konstanten Mikrowellen-Hochfrequenzsignals an die Übertragungsleitung (11) erzeugt wird, wobei das bekannte Prüfobjekt im Voraus bestätigt wurde, dass keine Fremdsubstanz gemischt ist; und
wenn sich der Referenzgradient von dem berechneten Gradienten unterscheidet, zu urteilen, dass eine Fremdsubstanz in dem Prüfobjekt (20) gemischt ist.

4. Zerstörungsfreies Detektionsvorrichtung nach Anspruch 3, weiter umfassend Positionspezifizierungsmittel (13), wobei das Positionspezifizierungsmittel (13) konfiguriert ist, um:
umzuwandeln, in eine Wellenform in einer Zeitdomäne, aus einer in einem Frequenzsignal erhaltenen Wellenform, das an der Übertragungsleitung (11) übertragen wird, wenn das Prüfobjekt (20), in dem es durch das Beurteilungsmittel (13) beurteilt wird, dass die Fremdsubstanz gemischt ist, innerhalb des elektromagnetischen Felds der Übertragungsleitung (11) angeordnet ist;
eine Amplitude und Position einer Nichtübereinstimmung zu detektieren, die der Fremdsubstanz entspricht, wobei die Detektion durch Berechnen des S-Parameters einschließlich des Eingangssignals und Ausgangssignals der Übertragungsleitung (11) und des reflektierten Signals durchgeführt wird, das zu dem Transceiver (12) zurückkehrt;
einen Abstand basierend auf einem Unterschied in Zeitantwort zwischen der Position, an der die Fremdsubstanz vorhanden ist, und der Position, an der die Fremdsubstanz in dem Prüfobjekt (20), das an der Übertragungsleitung (11) beurteilt wurde, die Fremdsubstanz aufzuweisen, fehlt, zu berechnen; und
die Position, an der die Fremdsubstanz in dem Prüfobjekt (20) gemischt ist, basierend auf dem Abstand zu bestimmen, der mit dem Unterschied in Zeitantwort des Hochfrequenzsignals berechnet wurde.

5. Zerstörungsfreie Detektionsvorrichtung, umfassend:
eine erste Übertragungsleitung (11a), deren Längsrichtung orthogonal zu Bewegungsrichtung eines Prüfobjekts (20) in Bewegung angeordnet ist;
eine zweite Übertragungsleitung (11b), die bezüglich der ersten Übertragungsleitung (11a) von der Bewegungsrichtung des Prüfobjekts (20) bei nachgeschaltet oder vorgeschaltet angeordnet ist, deren Längsrichtung schräg zu der Bewegungsrichtung des Prüfobjekts (20) angeordnet ist;
Übertragungsmittel (12a, 12b), das konfiguriert ist, um:
ein konstantes Mikrowellenband-Hochfrequenzsignal zu erzeugen und an die erste und zweite Übertragungsleitung (11a, 11b) zu übertragen;
außerhalb der Übertragungsleitung ein elektromagnetisches Feld zu erzeugen; und
die konstante Mikrowellenband-Hochfrequenzsignalausgabe von der ersten und zweiten Übertragungsleitung (11a, 11b) zu empfangen;
erstes Messmittel (12a), das konfiguriert ist, um:
durch Messen des konstanten Mikrowellenband-Hochfrequenzsignals erhaltenen ersten Wert zu erhalten, das von einer der Übertragungsleitungen (11a, 11b) ausgegeben wird, das an dem vorgeschaltet zwischen der ersten und zweiten Übertragungsleitung (11a, 11b) angeordnet ist und auch durch Empfangen durch die Übertragungsmittel (12a, 12b) gemessen wird, wobei die Vorrichtung angeordnet ist, um gleichzeitig eine Position eines Prüfobjekts (20) innerhalb des erzeugten elektromagnetischen Felds zu bewegen;
den ersten Wert, der zuerst gemessen wird, als einen ersten Referenzwert festzulegen; und
den ersten Wert, der an zweiter Stelle oder später gemessen wird, als einen ersten gemessenen Wert zur Beurteilung festzulegen;
erstes Gradientenberechnungsmittel (15), das konfiguriert ist, um einen Gradienten einer ersten geraden Linie bei einer vorher festgelegten 2D-Positionskoordinate basierend auf einem Unterschied zwischen dem ersten Referenzwert und dem ersten gemessenen Wert zur Beurteilung zu berechnen, wobei die vorher festgelegte 2D-Positionskoordinate eine 2D-Positionskoordinate ist, deren vertikale Achse eins ist von realem und imaginärem Teil eines S-Parameters des konstanten Mikrowellenband-Hochfrequenzsignals, ausgedrückt durch komplexe Notation oder eins von Phase und Amplitude, erhalten durch Berechnen des realen und imaginären Teils, und deren horizontale Achse der andere des realen und imaginären Teils oder die andere der Phase und Amplitude ist, wobei der S-Parameter ein Übertragungskoeffizient ist, der Spannungsverhältnis von Ausgangssignal zu Eingangssignal angibt;
erstes Beurteilungsmittel (15), das konfiguriert ist, um:
einen Referenzgradienten mit dem Gradienten der ersten geraden Linie zu vergleichen, der durch das erste Gradientenberechnungsmittel (15) berechnet wird, wobei der Referenzgradient der Gradient der geraden Linie an der vorher festgelegten 2D-Positionskoordinate ist, der im Voraus basierend auf dem gemessenen Wert des konstanten Mikrowellen-Hochfrequenzsignals von der ersten Übertragungslinie (11a) berechnet und gespeichert wurde, wenn ein bekanntes Prüfobjekt innerhalb des elektromagnetischen Felds angeordnet ist, das außerhalb der ersten Übertragungsleitung (11a) durch Übertragen des konstanten Mikrowellen-Hochfrequenzsignals an die erste Übertragungsleitung (11a) erzeugt wird, wobei das bekannte Prüfobjekt im Voraus bestätigt wurde, dass keine Fremdsubstanz gemischt ist; und
wenn sich der Referenzgradient von dem berechneten Gradienten der ersten geraden Linie unterscheidet, zu urteilen, dass eine Fremdsubstanz in dem Prüfobjekt (20) gemischt ist;
zweites Messmittel (12b), das konfiguriert ist, um:
durch Messen des konstanten Mikrowellenband-Hochfrequenzsignals erhaltenen zweiten Wert zu erhalten, das von einer der Übertragungsleitungen (11a, 11b) ausgegeben wird, das an dem nachgeschaltet zwischen der ersten und zweiten Übertragungsleitung (11a, 11b) angeordnet ist und auch durch Empfangen durch die Übertragungsmittel (12a, 12b) gemessen wird, wobei die Vorrichtung angeordnet ist, um gleichzeitig die Position des Prüfobjekts (20) innerhalb des erzeugten elektromagnetischen Felds zu bewegen;
den zweiten Wert, der zuerst gemessen wird, als einen zweiten Referenzwert festzulegen; und
den zweiten Wert, der an zweiter Stelle oder später gemessen wird, als einen zweiten gemessenen Wert zur Beurteilung festzulegen;
zweites Gradientenberechnungsmittel (15), das konfiguriert ist, um einen Gradienten einer zweiten geraden Linie an der vorher festgelegten 2D-Positionskoordinate basierend auf einem Unterschied zwischen dem zweiten Referenzwert und dem zweiten gemessenen Wert zur Beurteilung zu berechnen;
zweites Beurteilungsmittel (15), das konfiguriert ist, um:
den Referenzgradienten mit dem berechneten Gradienten der zweiten geraden Linie zu vergleichen; und
wenn sich der Referenzgradient von dem berechneten Gradienten der zweiten geraden Linie unterscheidet, zu beurteilen, dass die Fremdsubstanz in dem Prüfobjekt (20) gemischt ist; und
Intervallmessmittel (15), das konfiguriert ist, um ein Intervall zwischen Zeitpunkt, zu dem das erste Beurteilungsmittel (15) urteilt, dass die Fremdsubstanz in dem Prüfobjekt (20) gemischt ist, und Zeitpunkt, zu dem das zweite Beurteilungsmittel (15) urteilt, dass die Fremdsubstanz in dem Prüfobjekt (20) gemischt ist, zu messen,
wobei eine Position der in dem Prüfobjekt (20) gemischten Fremdsubstanz basierend auf dem Messintervall durch das Intervallmessmittel (15) ohne Beschädigung detektiert wird.

6. Zerstörungsfreie Detektionsvorrichtung, umfassend:
eine Übertragungsleitung (11); Übertragungsmittel (12), das konfiguriert ist, um:
ein Mikrowellenband-Hochfrequenzsignal oder ein synthetisches Hochfrequenzsignal zu erzeugen und an die Übertragungsleitung (11) zu übertragen, wobei das Hochfrequenzsignal durch Abtasten mit einem vorher festgelegten Frequenzbereich erzeugt wird, der an einer optimalen Frequenz zum Detektieren eines Prüfobjekts (20) zentriert ist, wobei das synthetische Hochfrequenzsignal aus einer optimalen Frequenz innerhalb des Frequenzbereichs und weiteren multiplen Frequenzelementen besteht;
multiple elektromagnetische Felder zu erzeugen, die multiplen Frequenzen des Hochfrequenzsignals entsprechen, wobei sich die erzeugten multiplen elektromagnetischen Felder zu einer Position erstrecken, an der das Prüfobjekt (20) platziert werden kann; und
das von der Übertragungsleitung (11) ausgegebene Hochfrequenzsignal zu empfangen;
Messmittel (12), das konfiguriert ist, um:
multiple Werte zu messen, die den multiplen Frequenzen des Hochfrequenzsignals entsprechen, die durch Messen des durch die Übertragungsleitung (12) empfangenen Hochfrequenzsignals erhalten wurden;
multiple Werte festzulegen, die zuerst als multiple Referenzwerte gemessen werden; und
multiple Werte, die an zweiter Stelle oder später gemessen werden, als multiple gemessene Werte zur Beurteilung festzulegen;
Gradientenberechnungsmittel (13), das konfiguriert ist, um multiple Gradienten von multiplen geraden Linien bei einer vorher festgelegten 2D-Positionskoordinate basierend auf einem Unterschied bei jeder Frequenz zwischen den Referenzwerten und den multiplen gemessenen Werten zur Beurteilung zu berechnen, wobei die vorher festgelegte 2D-Positionskoordinate eine 2D-Positionskoordinate ist, deren vertikale Achse eins ist von realem oder imaginärem Teil eines S-Parameters des Hochfrequenzsignals, ausgedrückt durch komplexe Notation oder eins von Phase und Amplitude, erhalten durch Berechnen des realen und imaginären Teils, und deren horizontale Achse der andere des realen und imaginären Teils oder die andere der Phase und Amplitude ist, wobei der S-Parameter ein Übertragungskoeffizient ist, der Spannungsverhältnis von Ausgangssignal zu Eingangssignal angibt; und
Beurteilungsmittel (13), das konfiguriert ist, um:
einen Referenzgradienten mit jedem der multiplen Gradienten der multiplen geraden Linien zu vergleichen, die durch das Gradientenberechnungsmittel (13) berechnet werden, wobei der Referenzgradient der Gradient der geraden Linie an der vorher festgelegten 2D-Positionskoordinate ist, der im Voraus basierend auf dem gemessenen Wert des Hochfrequenzsignals von der Übertragungslinie (11) berechnet und gespeichert wurde, wenn ein bekanntes Prüfobjekt innerhalb des elektromagnetischen Felds angeordnet ist, das außerhalb der Übertragungsleitung (11) durch Übertragen des Hochfrequenzsignals an die Übertragungsleitung (11) erzeugt wird, wobei das bekannte Prüfobjekt im Voraus bestätigt wurde, dass keine Fremdsubstanz gemischt ist; und
wenn sich der Referenzgradient von einem der berechneten multiplen Gradienten unterscheidet, zu urteilen, dass eine Fremdsubstanz in dem Prüfobjekt (20) gemischt ist.

7. Zerstörungsfreie Detektionsvorrichtung nach einem der Ansprüche 3 bis 6,
wobei der gemessene Wert des Hochfrequenzsignals Wert des realen und imaginären Teils ist.

8. Computerprogramm, umfassend Anweisungen, die, wenn von einer zerstörungsfreien Detektionsvorrichtung nach einem der Ansprüche 3 bis 5, und Anspruch 7 wenn abhängig von einem der Ansprüche 3 bis 5, die zerstörungsfreie Detektionsvorrichtung veranlassen, das zerstörungsfreie Detektionsverfahren nach Anspruch 1 oder 2 auszuführen.

## Revendications

1. Procédé de détection non destructive, comprenant :
une étape de transmission de signal (S1) pour transmettre un signal à haute fréquence constante en bande micro-ondes à une ligne de transmission (11) et générer, à l'extérieur de la ligne de transmission (11), un champ électromagnétique à une position à laquelle un objet de test (20) est au moins présent ;
une étape de mesure de référence (S2, S3) pour mesurer le signal à haute fréquence constante en bande micro-ondes transmis par l'intermédiaire de la ligne de transmission (11) et régler une valeur qui est mesurée en premier en tant qu'une valeur de référence ;
une étape de mesure (S4, S5) pour mesurer le signal à haute fréquence constante en bande micro-ondes transmis par l'intermédiaire de la ligne de transmission (11) pendant le déplacement de la position de l'objet de test (20) et obtenir une valeur qui est mesurée en deuxième ou ultérieurement ;
une étape de calcul de gradient (S6) pour calculer un gradient d'une ligne droite à une coordonnée de position 2D prédéterminée sur la base d'une différence entre la valeur de référence et la valeur qui est mesurée en deuxième ou ultérieurement, dans lequel la coordonnée de position 2D prédéterminée est une coordonnée de position 2D dont un axe vertical est l'une parmi une partie réelle et une partie imaginaire d'un paramètre S du signal à haute fréquence constante en bande micro-ondes exprimée par une notation complexe ou l'une parmi une phase et une amplitude obtenues par le calcul de la partie réelle et de la partie imaginaire et dont un axe horizontal est l'autre parmi la partie réelle et la partie imaginaire ou l'autre parmi la phase et l'amplitude, dans lequel le paramètre S est un coefficient de transmission indiquant un rapport de tension d'un signal de sortie sur un signal d'entrée ; et
une étape de jugement (S7) comprenant les étapes suivantes :
la comparaison d'un gradient de référence au gradient qui est calculé à l'étape de calcul de gradient, le gradient de référence est le gradient de la ligne droite à la coordonnée de position 2D prédéterminée qui a été calculé à l'avance sur la base de la valeur mesurée du signal à haute fréquence constante en bande micro-ondes à partir de la ligne de transmission (11) lorsque le signal à haute fréquence constante en bande micro-ondes est transmis à la ligne de transmission (11) et également le champ électromagnétique de la ligne de transmission (11) est généré à une position à laquelle un objet de test connu existe, l'objet de test connu a été confirmé à l'avance qu'aucune substance étrangère n'est mélangée ; et
lorsque le gradient de référence est différent du gradient calculé, le jugement qu'une substance étrangère est mélangée dans l'objet de test (20).

2. Procédé de détection non destructive selon la revendication 1,
dans lequel les valeurs mesurées à l'étape de mesure de valeur de référence (S2, S3) et l'étape de mesure (S4, S5), et la valeur mesurée du signal à haute fréquence constante en bande micro-ondes utilisée pour le calcul du gradient de référence sont des valeurs de la partie réelle et de la partie imaginaire.

3. Dispositif de détection non destructive, comprenant :
une ligne de transmission (11) ;
un moyen émetteur-récepteur (12) qui est configuré pour :
transmettre un signal à haute fréquence constante en bande micro-ondes à la ligne de transmission (11) ;
générer, à l'extérieur de la ligne de transmission (11), un champ électromagnétique ; et
recevoir le signal à haute fréquence constante en bande micro-ondes délivré depuis la ligne de transmission (11) ;
un moyen de mesure (12) qui est configuré pour :
mesurer le signal à haute fréquence constante en bande micro-ondes transmis par l'intermédiaire de la ligne de transmission (11), le dispositif étant agencé pour déplacer simultanément une position d'un objet de test (20) à l'intérieur du champ électromagnétique généré ;
régler une valeur qui est mesurée en premier en tant qu'une valeur de référence ; et
régler une valeur qui est mesurée en deuxième ou ultérieurement en tant qu'une valeur mesurée de jugement ;
un moyen de calcul de gradient (13) qui est configuré pour calculer un gradient d'une ligne droite à une coordonnée de position 2D prédéterminée sur la base d'une différence entre la valeur de référence et la valeur de jugement, dans lequel la coordonnée de position 2D prédéterminée est une coordonnée de position 2D dont un axe vertical est l'une parmi une partie réelle et une partie imaginaire d'un paramètre S du signal à haute fréquence constante en bande micro-ondes exprimée par une notation complexe ou l'une parmi une phase et une amplitude obtenues par le calcul de la partie réelle et de la partie imaginaire et dont un axe horizontal est l'autre parmi la partie réelle et la partie imaginaire ou l'autre parmi la phase et l'amplitude, dans lequel le paramètre S est un coefficient de transmission indiquant un rapport de tension d'un signal de sortie sur un signal d'entrée ;
et un moyen de jugement (13) qui est configuré pour :
comparer un gradient de référence au gradient qui est calculé par le moyen de calcul de gradient (13), le gradient de référence est le gradient de la ligne droite à la coordonnée de position 2D prédéterminée qui a été calculé et mémorisé à l'avance sur la base de la valeur mesurée du signal à haute fréquence constante en bande micro-ondes à partir de la ligne de transmission (11) lorsqu'un objet de test connu est agencé à l'intérieur du champ électromagnétique qui est généré à l'extérieur de la ligne de transmission (11) par la transmission du signal à haute fréquence constante en bande micro-ondes à la ligne de transmission (11), l'objet de test connu a été confirmé à l'avance qu'aucune substance étrangère n'est mélangée ; et
lorsque le gradient de référence est différent du gradient calculé, juger qu'une substance étrangère est mélangée dans l'objet de test (20).

4. Dispositif de détection non destructive selon la revendication 3, comprenant en outre un moyen de spécifications de position (13), dans lequel le moyen de spécifications de position (13) est configuré pour :
transformer en une forme d'onde dans un domaine de temps à partir d'une forme d'onde obtenue dans un signal de fréquence qui est transmis sur la ligne de transmission (11) lorsque l'objet de test (20), dans lequel il est jugé par le moyen de jugement (13) que la substance étrangère est mélangée, est agencé à l'intérieur du champ électromagnétique de la ligne de transmission (11) ;
détecter une amplitude et une position d'une discordance correspondant à la substance étrangère, dans lequel la détection est réalisée par le calcul du paramètre S incluant le signal d'entrée et le signal de sortie de la ligne de transmission (11), et le signal réfléchi qui revient vers l'émetteur-récepteur (12) ;
calculer une distance sur la base d'une différence de réponse temporelle entre la position à laquelle la substance étrangère est présente et la position à laquelle la substance étrangère est absente dans l'objet de test (20) qui a été jugé comme présentant la substance étrangère sur la ligne de transmission (11) ; et
déterminer la position à laquelle la substance étrangère est mélangée dans l'objet de test (20) sur la base de la distance qui a été calculée avec la différence de réponse temporelle du signal à haute fréquence.

5. Dispositif de détection non destructive, comprenant :
une première ligne de transmission (11a) dont une direction longitudinale est agencée orthogonalement à une direction de déplacement d'un objet de test (20) en mouvement ;
une seconde ligne de transmission (11b), qui est agencée en aval ou en amont de la direction de déplacement de l'objet de test (20) par rapport à la première ligne de transmission (11a), dont une direction longitudinale est agencée obliquement à la direction de déplacement de l'objet de test (20) ;
un moyen de transmission (12a, 12b) qui est configuré pour :
générer et transmettre un signal à haute fréquence constante en bande micro-ondes aux première et seconde lignes de transmission (11a, 11b) ;
générer, à l'extérieur de la ligne de transmission, un champ électromagnétique ; et
recevoir le signal à haute fréquence constante en bande micro-ondes délivré depuis les première et seconde lignes de transmission (11a, 11b) ;
un premier moyen de mesure (12a) qui est configuré pour :
obtenir une première valeur obtenue par la mesure du signal à haute fréquence constante en bande micro-ondes qui est délivré depuis l'une des lignes de transmission (11a, 11b) qui est agencé en amont entre les première et seconde lignes de transmission (11a, 11b) et également mesuré par la réception par le moyen de transmission (12a, 12b), le dispositif étant agencé pour déplacer simultanément une position d'un objet de test (20) à l'intérieur du champ électromagnétique généré ;
régler la première valeur qui est mesurée en premier en tant qu'une première valeur de référence ; et
régler la première valeur qui est mesurée en deuxième ou ultérieurement en tant qu'une première valeur mesurée de jugement ;
un premier moyen de calcul de gradient (15) qui est configuré pour calculer un gradient d'une première ligne droite à une coordonnée de position 2D prédéterminée sur la base d'une différence entre la première valeur de référence et la première valeur mesurée de jugement, dans lequel la coordonnée de position 2D prédéterminée est une coordonnée de position 2D dont un axe vertical est l'une parmi une partie réelle et une partie imaginaire d'un paramètre S du signal à haute fréquence constante en bande micro-ondes exprimée par une notation complexe ou l'une parmi une phase et une amplitude obtenues par le calcul de la partie réelle et de la partie imaginaire et dont un axe horizontal est l'autre parmi la partie réelle et la partie imaginaire ou l'autre parmi la phase et l'amplitude, dans lequel le paramètre S est un coefficient de transmission indiquant un rapport de tension d'un signal de sortie sur un signal d'entrée ;
un premier moyen de jugement (15) qui est configuré pour :
comparer un gradient de référence au gradient de la première ligne droite qui est calculé par le premier moyen de calcul de gradient (15), le gradient de référence est le gradient de la ligne droite à la coordonnée de position 2D prédéterminée qui a été calculé et mémorisé à l'avance sur la base de la valeur mesurée du signal à haute fréquence constante en bande micro-ondes à partir de la première ligne de transmission (11a) lorsqu'un objet de test connu est agencé à l'intérieur du champ électromagnétique qui est généré à l'extérieur de la première ligne de transmission (11a) par la transmission du signal à haute fréquence constante en bande micro-ondes à la première ligne de transmission (11a), l'objet de test connu a été confirmé à l'avance qu'aucune substance étrangère n'est mélangée ; et
lorsque le gradient de référence est différent du gradient calculé de la première ligne droite, juger qu'une substance étrangère est mélangée dans l'objet de test (20) ;
un second moyen de mesure (12b) qui est configuré pour :
obtenir une seconde valeur obtenue par la mesure du signal à haute fréquence constante en bande micro-ondes qui est délivré depuis l'une des lignes de transmission (11a, 11b) qui est agencé en aval entre les première et seconde lignes de transmission (11a, 11b) et également mesuré par la réception par le moyen de transmission (12a, 12b), le dispositif étant agencé pour déplacer simultanément la position de l'objet de test (20) à l'intérieur du champ électromagnétique généré ;
régler la seconde valeur qui est mesurée en premier en tant qu'une seconde valeur de référence ; et
régler la seconde valeur qui est mesurée en deuxième ou ultérieurement en tant qu'une seconde valeur mesurée de jugement ;
un second moyen de calcul de gradient (15) qui est configuré pour calculer un gradient d'une seconde ligne droite à la coordonnée de position 2D prédéterminée sur la base d'une différence entre la seconde valeur de référence et la seconde valeur mesurée de jugement ;
un second moyen de jugement (15) qui est configuré pour :
comparer le gradient de référence au gradient calculé de la seconde ligne droite ; et
lorsque le gradient de référence est différent du gradient calculé de la seconde ligne droite, juger que la substance étrangère est mélangée dans l'objet de test (20) ; et
un moyen de mesure d'intervalle (15) qui est configuré pour mesurer un intervalle entre un timing auquel le premier moyen de jugement (15) juge que la substance étrangère est mélangée dans l'objet de test (20) et un timing auquel le second moyen de jugement (15) juge que la substance étrangère est mélangée dans l'objet de test (20),
dans lequel une position de la substance étrangère mélangée dans l'objet de test (20) est détectée sans aucune détérioration sur la base de l'intervalle mesuré par le moyen de mesure d'intervalle (15).

6. Dispositif de détection non destructive, comprenant :
une ligne de transmission (11) ; un moyen de transmission (12) qui est configuré pour :
générer et transmettre, à la ligne de transmission (11), un signal à haute fréquence en bande micro-ondes ou un signal à haute fréquence synthétique, le signal à haute fréquence est généré par balayage avec une plage de fréquences prédéterminée centrée sur une fréquence optimale pour détecter un objet de test (20), le signal à haute fréquence synthétique se composant d'une fréquence optimale à l'intérieur de la plage de fréquences et d'autres éléments de fréquences multiples ;
générer de multiples champs électromagnétiques correspondant à de multiples fréquences du signal à haute fréquence, les multiples champs électromagnétiques générés s'étendant jusqu'à une position à laquelle l'objet de test (20) peut être placé ; et
recevoir le signal à haute fréquence délivré depuis la ligne de transmission (11) ;
un moyen de mesure (12) qui est configuré pour :
mesurer de multiples valeurs correspondant aux multiples fréquences du signal à haute fréquence obtenues par la mesure du signal à haute fréquence reçu par le moyen de transmission (12) ;
régler de multiples valeurs qui sont mesurées en premier en tant que multiples valeurs de référence ; et
régler de multiples valeurs qui sont mesurées en deuxième ou ultérieurement en tant que multiples valeurs mesurées de jugement ;
un moyen de calcul de gradient (13) qui est configuré pour calculer de multiples gradients de multiples lignes droites à une coordonnée de position 2D prédéterminée sur la base d'une différence, dans chaque fréquence, entre les valeurs de référence et les multiples valeurs mesurées de jugement, dans lequel la coordonnée de position 2D prédéterminée est une coordonnée de position 2D dont un axe vertical est l'une parmi une partie réelle et une partie imaginaire d'un paramètre S du signal à haute fréquence exprimée par une notation complexe ou l'une parmi une phase et une amplitude obtenues par le calcul de la partie réelle et de la partie imaginaire et dont un axe horizontal est l'autre parmi la partie réelle et la partie imaginaire ou l'autre parmi la phase et l'amplitude, dans lequel le paramètre S est un coefficient de transmission indiquant un rapport de tension d'un signal de sortie sur un signal d'entrée ; et
un moyen de jugement (13) qui est configuré pour :
comparer un gradient de référence à chacun des multiples gradients des multiples lignes droites qui sont calculés par le moyen de calcul de gradient (13), le gradient de référence est le gradient de la ligne droite à la coordonnée de position 2D prédéterminée qui a été calculé et mémorisé à l'avance sur la base de la valeur mesurée du signal à haute fréquence à partir de la ligne de transmission (11) lorsqu'un objet de test connu est agencé à l'intérieur du champ électromagnétique qui est généré à l'extérieur de la ligne de transmission (11) par la transmission du signal à haute fréquence à la ligne de transmission (11), l'objet de test connu a été confirmé à l'avance qu'aucune substance étrangère n'est mélangée ; et
lorsque le gradient de référence est différent de l'un quelconque des multiples gradients calculés, juger qu'une substance étrangère est mélangée dans l'objet de test (20).

7. Dispositif de détection non destructive selon l'une quelconque des revendications 3 à 6,
dans lequel la valeur mesurée du signal à haute fréquence est une valeur des parties réelle et imaginaire.

8. Programme informatique comprenant des instructions qui, lorsqu'elles sont exécutées par un dispositif de détection non destructive selon l'une quelconque des revendications 3 à 5, et la revendication 7 dépendant de l'une quelconque des revendications 3 à 5, amènent le dispositif de détection non destructive à exécuter le procédé de détection non destructive selon la revendication 1 ou 2.
